# EUROPÄISCHE PATENTSCHRIFT

(11) **EP 1 440 315 B1**
(45) Veröffentlichungstag und Bekanntmachung des Hinweises auf die Patenterteilung: **24.01.2007**
(21) Anmeldenummer: 02767289.8
(22) Anmeldetag: 31.07.2002
(51) Int. Cl.: G01N 33/564, G01N 33/58

(54) **NACHWEIS HUMANER AUTO-ANTIKÖRPER**
IDENTIFICATION OF HUMAN AUTO-ANTIBODIES
IDENTIFICATION D'AUTO-ANTICORPS HUMAINS

(30) Priorität: 01.08.2001 DE 10137288
(43) Veröffentlichungstag der Anmeldung: 28.07.2004
(73) Patentinhaber: Wolfrum, Jürgen, 69120 Heidelberg (DE); Sauer, Markus, 68124 Heidelberg (DE)
(72) Erfinder: Wolfrum, Jürgen, 69120 Heidelberg (DE); Sauer, Markus, 68124 Heidelberg (DE)
(74) Vertreter: Köllner, Malte
(86) Internationale Anmeldenummer: PCT/EP2002/008532
(87) Internationale Veröffentlichungsnummer: WO 2003/014742

(56) Entgegenhaltungen:
- WO-A-87/07385
- WEL A-P ET AL: "ANTIBODY-MEDIATED FLUORESCENCE ENHANCEMENT BASED ON SHIFTING THE INTRAMOLECULAR DIMER MONOMER EQUILIBRIUM OF FLUORESCENT DYES" ANALYTICAL CHEMISTRY, AMERICAN CHEMICAL SOCIETY. COLUMBUS, US, Bd. 66, Nr. 9, Mai 1994 (1994-05), Seiten 1500-1506, XP002937508 ISSN: 0003-2700 in der Anmeldung erwähnt
- GEOGHEGAN KIERAN F ET AL: "Dye-pair reporter systems for protein-peptide molecular interactions." BIOCONJUGATE CHEMISTRY, Bd. 11, Nr. 1, Januar 2000 (2000-01), Seiten 71-77, XP002247287 ISSN: 1043-1802

## Beschreibung

Veränderungen in den Genen des Zellkerns können eine "normale" Zelle zur Krebszelle werden lassen. DNA-Schädigungen in so genannten Tumorsuppressor-Genen sind dabei von besonderer Bedeutung, da die von den Tumorsuppressor-Genen kodierten Proteine das unkontrollierte Wachstum einer Zelle, also die Entstehung des Tumors, verhindern.

Das p53-Tumorsuppressor-Gen (kurz p53), das von entscheidender Bedeutung in der Regulation des Zellzyklus ist, besitzt besondere Relevanz, da in 60-80% aller Tumore Mutationen in diesem Gen nachgewiesen werden können, und zwar unabhängig von der Art und Herkunft des Tumors. Es wurden in den letzten Jahren eine Vielzahl von Mutationen in völlig verschiedenen Bereichen des p53-Gens beschrieben; alle diese Mutationen haben aber letztendlich den gleichen Effekt, nämlich den Verlust der tumorsuppressiven Eigenschaften des vom p53-Tumorsuppressor-Gen kodierten p53-Proteins. Dabei führen die meisten dieser Mutationen zu einer Verlängerung der biologischen Halbwertszeit des p53-Proteins von ca. 10 bis 20 Minuten auf bis zu 48 Stunden beim mutanten p53-Protein. Dies bewirkt eine starke Akkumulation von mutanten p53-Proteinen in der Zelle.

Wegen der kurzen Halbwertszeit und der damit verbundenen geringen Menge des Proteins in normalen oder gesunden Zellen ist der Nachweis des p53-Proteins im Serum oder anderen Körperflüssigkeiten schwierig und nur in sehr wenigen Fällen beschrieben. Dies liegt auch an der Instabilität des p53-Proteins und der Tatsache, dass es in erster Linie ein Kernprotein ist, d. h. im Zellkern vorkommt.

Mutantes p53-Protein kann jedoch, da es ein tumorspezifisches Antigen ist, das Immunsystem aktivieren. So konnte gezeigt werden, dass viele Tumorpatienten p53-Auto-Antikörper gegen die abnormal hohe Konzentration des mutanten p53-Proteins entwickeln.

Allgemein ist ein Auto-Antikörper ein vom Immunsystem produzierter Antikörper gegen ein körpereigenes Antigen, also gegen einen im Körper selbst produzierten Stoff, dessen Produktion in der Regel nur bei Vorliegen eines abnormen Zustands erfolgt, z. B. wenn ein Tumor entstanden ist. Insofern erfüllen Auto-Antikörper häufig die Funktion von Tumormarkern.

Das Immunsystem reagiert dabei nicht deshalb, weil das p53-Protein mutiert ist, sondern weil es in unüblich hoher Konzentration vorhanden ist. Dabei ist diese für den Körper "unüblich hohe Konzentration" immer noch sehr gering.

Von besonderem Interesse ist die Tatsache, dass 100% aller Patienten, bei denen bisher p53-Auto-Antikörper nachgewiesen werden konnten, eine durch andere Verfahren gesicherte Tumorerkrankung aufwiesen und keinerlei Korrelation mit den klassischen Tumormarkern existiert.

Daher ist eine Bestimmung von p53-Auto-Antikörpern auch bei Patienten sinnvoll, bei denen kein sonstiger Tumormarker festgestellt werden konnte.

Wichtig ist ferner, dass p53-Auto-Antikörper bei praktisch allen Tumorerkrankungen nachgewiesen werden konnten, wobei der Prozentsatz positiver Befunde bei den verschiedenen Tumorarten variiert.

Auch der Einsatz der p53-Antikörper-Bestimmung bei der Nachsorge von behandelten Tumorerkrankungen ist sinnvoll, denn es konnte gezeigt werden, dass das Auftreten von p53-Auto-Antikörpern zeitlich oft deutlich vor den klassischen Tumormarkern nachgewiesen werden kann.

Bisher werden Auto-Antikörper mit Hilfe eines herkömmlichen ELISA-Tests bestimmt (ELISA = enzyme linked immunosorbant assay). Beim ELISA-Test befindet sich das häufig sehr teure Protein (Antigen) auf einer ELISA-Platte. Die Probe des Patienten wird auf die Platte gegeben und eine Bindung der Auto-Antikörper an das Protein findet statt, sofern Auto-Antikörper in der Probe vorhanden sind. Anschließend wird ein Sekundärantikörper mit einem daran gekoppelten farbreaktionsfähigen Enzym zugefügt. Anschließend werden ungebundene Substanzen durch einen Spülschritt entfernt. Viele Auto-Antikörper gehören in die Gruppe der IgG-Antikörper. Als Sekundärantikörper kann in solchen Fällen ein Anti-IgG-Antikörper der Maus genommen werden. Das angekoppelte farbreaktionsfähige Enzym kann eine Farbreaktion katalysieren, deren Produkte durch Absorption in einem Spektrometer nachgewiesen werden können.

In einem anderen bekannten Verfahren zum Nachweis von Antikörpern [Wei A. P.; Blumenthal D.K.; Herron, J.N.; Anal. Chem. 1994, Band 66, Seiten 1500-1506] wird ein Epitop mit zwei Farbstoffen, Fluorescein und Tetramethylrhodamin, gefärbt. Im ungebundenen Zustand nimmt das Epitop eine Konformation ein, bei der die beiden Farbstoffe in räumliche Nähe zueinander geraten. Die Fluoreszenz des Fluoresceins wird dann durch das Tetramethylrhodamin gelöscht. Die Bindung des Epitops an einen Antikörper führt zu einer Konformationsänderung des Epitops. Der Abstand zwischen Fluorescein und Tetramethylrhodamin nimmt dabei zu und die Löschung der Fluoreszenz nimmt ab. Die Bindung führt somit zu einem Anstieg der Fluoreszenz, der zum Nachweis des Antikörpers genutzt werden kann. Dieses Verfahren hat jedoch den Nachteil, dass ein Epitop relativ aufwändig mit zwei Farbstoffen markiert werden muss. Dabei ändern sich auch die Eigenschaften des Epitops in erheblichem Maße. Insbesondere wird dadurch die Affinität zwischen Epitop und Antikörper verringert. Daraus folgt wiederum eine Verringerung der Empfindlichkeit des Nachweisverfahrens.

Auch WO 87 07385 oder GEOGHEGAN K et al. ('Dye-pair reporter systems for proteinpeptide molecular interactions.' BIOCONJUGATE CHEMISTRY, Bd. 11, Nr. 1, Januar 2000, Seiten 71-77) beschreiben Verfahren, die auf der Verwendung von Peptiden beruhen, die mit 2 Fluorophoren markiert wurden.

Aufgabe der Erfindung ist es, ein Verfahren anzugeben, das den Nachweis von Auto-Antikörpern vereinfacht.

Diese Aufgabe wird durch die Erfindung gemäß dem unabhängigen Anspruch gelöst. Vorteilhafte Weiterbildungen der Erfindung sind in den Unteransprüchen gekennzeichnet.

Erfindungsgemäß wird zunächst ein Antikörper derart gewählt, dass er an mindestens ein Epitop eines Antigens binden kann. Ein Epitop ist ein Peptid mit einer Länge von 1 bis etwa 40 Aminosäuren. Als Epitop werden diejenigen Regionen eines Proteins bezeichnet, an die ein Antikörper spezifisch bindet, mit anderen Worten, die dem Antikörper als Erkennungsregion dienen.

Ein Epitop und ein Fluorophor werden derart gewählt, dass das Epitop mindestens einen Tryptophan-Rest (Quencher) aufweist, der die Lumineszenz des Fluorophors bei hinreichender räumlicher Nähe zwischen dem Fluorophor und dem Tryptophan-Rest quenchen kann.

Mit Lumineszenz werden allgemein alle Arten der Lichtstreuung durch Moleküle bzw. Partikel bezeichnet, seien sie spontan, wie bei der Raman-Streuung, oder verzögert, wie bei der Fluoreszenz oder Phosphoreszenz.

Das gewählte Epitop kann auch durch zusätzliche Aminosäuren verlängert oder modifiziert werden. Wichtig ist, dass der Antikörper das Epitop noch als solches erkennt.

Als Farbstoff kann prinzipiell jedes Rhodamin oder Oxazin gewählt werden. Vorzugsweise wird der Oxazin-Farbstoff MR121 eingesetzt [M. Sauer, J. Arden-Jacob, K. H. Drexhage, F. Göbel, U. Lieberwirth, K. Mühlegger, R. Müller, J. Wolfrum, C. Zander (1998) Time-resolved identification of individual mononucleotide molecules in aqueous solution with pulsed semiconductor lasers, Bioimaging 6, 14-24.] [M. Sauer, K. H. Drexhage, U. Lieberwirth, R. Müller, S. Nord, C. Zander (1998) Dynamics of electron-transfer reactions between xanthene dyes and DNA nucleotides monitored on the single molecule level, Chem. Phys. Lett. 284, 153-163.], da seine Fluoreszenz sich besonders gut durch Tryptophan löschen lässt.

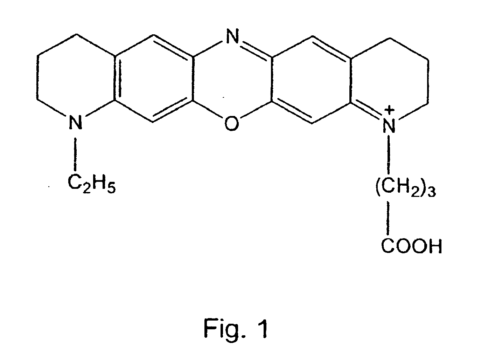

Die Struktur von MR121 ist in Fig. 1 dargestellt.

Die vorzugsweise zur Anwendung kommenden Rhodamine oder Oxazine sind so genannte "rote" Farbstoffe, d. h. sie absorbieren und emittieren Licht im roten Spektralbereich. Der Einsatz eines roten Farbstoffs zusammen mit einer Anregung im roten Bereich des sichtbaren Spektrums reduziert die Eigenfluoreszenz biologischer Proben erheblich, die stets eine Reihe von Substanzen enthalten, die im gelben, grünen und blauen Spektralbereich des sichtbaren Lichts absorbieren und emittieren. Dadurch wird das Hintergrundsignal deutlich reduziert und somit die Empfindlichkeit deutlich gesteigert.

Die Epitope werden mit dem ausgewählten Farbstoff markiert. Die Markierung erfolgt N- oder C-terminal oder innerhalb der Aminosäuresequenz an z. B. einem Lysinrest.

Das markierte Epitop faltet sich. Die hydrophobe Wechselwirkung zwischen dem relativ hydrophoben Farbstoff und der hydrophoben Aminosäure Tryptophan führt in wässrigen Medien zu einer räumlichen Annäherung zwischen dem Farbstoff und dem Tryptophan-Rest. Aufgrund der räumlichen Nähe kommt es zu einer Wechselwirkung zwischen dem Farbstoff und dem Tryptophan-Rest. Dadurch kommt es zu einer Löschung der Fluoreszenz des Farbstoffs, aller Wahrscheinlichkeit nach durch photoinduzierten Elektronentransfer zwischen dem Farbstoff und dem Tryptophan-Rest.

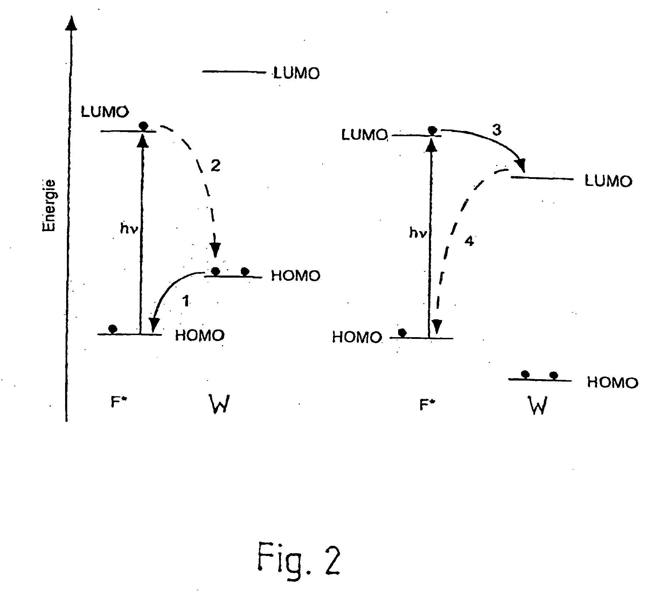

Im Folgenden sei der photoinduzierte Elektronentransfer anhand von Fig. 2 kurz erläutert. Dargestellt ist die Fluoreszenzlöschung eines angeregten Farbstoff-Moleküls F* durch einen Tryptophan-Rest W. Die schwarzen Kreise repräsentieren Elektronen. Es sind jeweils das HOMO (highest occupied molecular orbital) und das LUMO (lowest unoccupied molecular orbital) eingezeichnet. Das HOMO ist das energetisch höchste, im elektronischen Grundzustand besetzte Molekülorbital. Das LUMO ist das energetisch niedrigste, im elektronischen Grundzustand unbesetzte Molekülorbital; es ist i. d. R. das Molekülorbital, das im ersten angeregten Zustand besetzt wird.

Prinzipiell gibt es zwei Möglichkeiten der Fluoreszenzlöschung durch photoinduzierten Elektronentransfer. Im in Fig. 2 links dargestellten Fall wirkt der Tryptophan-Rest W als Elektronenspender (Donor). Nach Anregung des Fluorophors F* geht ein Elektron vom doppelt besetzten HOMO des Tryptophan-Rests W zum nun einfach besetzen HOMO des Fluorophors F* über (1). Es kommt zu einer Reduktion des angeregten Fluorophors F* durch den Tryptophan-Rest W. Das Elektron im LUMO des Fluorophors F* kann anschließend zum nun einfach besetzten HOMO des Tryptophan-Rests W übergehen (2). Dieser Fall tritt zwischen roten Farbstoffen und einem Tryptophan-Rest W auf.

Im in Fig. 2 rechts dargestellten Fall wirkt der Tryptophan-Rest W als Elektronenakzeptor (Akzeptor). Aus dem einfach besetzten LUMO des angeregten Fluorophors F* geht das dort befindliche Elektron zum unbesetzten LUMO des Tryptophan-Rests W über (3). Es kommt zu einer Oxidation des angeregten Fluorophors F* durch den Tryptophan-Rest W. Das Elektron im LUMO des Tryptophan-Rests W kann anschließend zum HOMO des Fluorophors zurückkehren (4).

In beiden Fällen kann das Elektron nach dem Elektronentransfer nicht mehr aus dem LUMO des angeregten Fluorophors F* durch Aussenden eines Photons in das HOMO zurückkehren. Der erste angeregte Zustand wurde strahlungslos deaktiviert. Die Fluoreszenz ist gelöscht.

Aufgrund der Fluoreszenzlöschung kommt es somit zu einer Verringerung der Fluoreszenzintensität des markierten Epitops. Im Falle des Farbstoffs MR121 beträgt die Verringerung z. B. ca. 63 %.

Mischt man jedoch die Antikörper und das markierte Epitop in der Lösung, so können Epitop und Antikörper binden. Beobachtet man die Lumineszenz der Lösung in einem Detektionsvolumen, so kann eine Änderung der Lumineszenz nach dem Mischen von Antikörper und markiertem Epitop festgestellt werden.

Bindet der Antikörper an das Epitop, initialisiert die Bindung eine Konformationsänderung im Epitop. Der Tryptophan-Rest kommt dabei in der Regel tief innerhalb der hydrophoben Bindungstasche des Antikörpers zu liegen [Kussie P.H., Gorina, S.; Marechal, V.; Elenbaas, B.; Moreau, J.; Levine, A.J.; Pavelitch, N.P., Science 1996, Band 274, Seiten 948-953], während der Farbstoff außen bleibt. Dadurch geht der räumlich nahe Kontakt des Farbstoffs zum Tryptophan-Rest und damit die Fluoreszenzlöschung verloren.

Es resultiert ein Fluoreszenzanstieg, der auf die Bindung zwischen Epitop und Antikörper hinweist. Damit weist ein Fluoreszenzanstieg auf die Anwesenheit von Antikörpern hin. Die Stärke des Fluoreszenzanstiegs erlaubt eine quantitative Bestimmung der Konzentration der Antikörper.

Da kein Spülvorgang oder ähnliche weitere Schritte erforderlich sind (wie beim bekannten ELISA-Test), handelt es sich bei dem erfindungsgemäßen Nachweisverfahren um einen "homogenen Assay". Er ist einfach und preisgünstig. Es wird nur ein farbstoffmarkiertes Peptid benötigt. Zudem bindet der Antikörper schneller an das Antigen, die Inkubationszeit kann daher von mehreren Stunden (beim ELISA-Test) auf ca. 1 h gesenkt werden. Hinzu kommt, dass Bindungen in Lösung allgemein spezifischer sind als an Oberflächen, bei denen stets auch unspezifische Adsorption stattfindet.

Bei der Durchführung des erfindungsgemäßen Nachweisverfahrens in einer normalen Küvette ist die Empfindlichkeit auf ein mittleres Niveau begrenzt. Die Fluoreszenz des freien Epitops ist gegenüber der Fluoreszenz des gebundenen Epitops auf ca. die Hälfte oder ein Drittel reduziert, d. h. ein zwei- bis dreifacher Anstieg der Fluoreszenz kann im Falle einer Bindung beobachtet werden. Die erreichbare Empfindlichkeit liegt bei ca. 10^-8 bis 10^-9 Mol pro Liter.

Wenn man das Verfahren jedoch auf Einzelmolekülebene anwendet, wird es wesentlich empfindlicher.
(a) Wenn man die Signale einzelner Bindungskomplexe zwischen markiertem Epitop und Antikörper detektiert, kann man effizient zwischen freien und gebundenen Signalen diskriminieren, insbesondere, wenn die Anregungsleistung so weit gesenkt wird, dass die freien Epitope mit der gelöschten Fluoreszenz im Mittel nur kleine Signale geben. Damit kann man mit einem simplen Schwellenwert, der festlegt, ab welcher Signalstärke ein Einzelmolekülsignal (Burst) akzeptiert wird, hochempfindlich Bindungskomplexe bis 10^-12 Mol/l nachweisen.
(b) Bei FCS-Messungen auf Einzelmolekülebene leuchten die gebundenen Epitope stärker und tragen damit mehr zum Signal bei.

Gegenüber Versuchen in einer einfachen Küvette in einem Spektrometer kann mit Hilfe eines konfokalen Mikroskops und der Arbeit auf Einzelmolekül-Niveau unter Ausnutzung sämtlicher Parameter eine Steigerung der Empfindlichkeit um mehr als einen Faktor 60 erreicht werden.

Eine weitere Steigerung der Empfindlichkeit kann durch eine verbesserte Diskriminierung zwischen den Signalen von Bindungskomplexen und den Signalen von freien Epitopen erreicht werden. Zur Diskriminierung bietet sich neben der Intensität (welche und wie viele Bursts liegen oberhalb einer vorgegebenen Signalschwelle), auch die Fluoreszenzlebensdauer des Farbstoffs an, die durch die Wechselwirkung mit dem Tryptophan-Rest verkürzt wird, also beim freien Epitop gegenüber dem Bindungskomplex verkürzt ist.

Auch die Diffusionszeit kann zur Diskriminierung genutzt werden. Das freie farbstoffmarkierte Epitop hat eine Diffusionszeit von etwa 0,3 ms durch ein Beobachtungsvolumen mit einem Durchmesser von etwa 1 µm. Der Bindungskomplex ist etwa 70 mal so schwer wie das freie farbstoffmarkierte Epitop. Der Bindungskomplex hat daher eine kleinere Diffusionskonstante und damit eine größere Diffusionszeit, nämlich etwa 1 ms. Die Diffusionszeit lässt sich beispielsweise mittels einer FCS-Messung oder bei jedem Durchtritt des Farbstoffs durch das Beobachtungsvolumen beobachten.

Schließlich kann auch die Fluoreszenz-Anisotropie sowie der Fluoreszenz-Anisotropie-Zerfall von jedem einzelnen Signal zur Diskriminierung herangezogen werden.

All diese Parameter ändern sich nur leicht vom freien zum gebundenen Zustand des Epitops. Die besten Ergebnisse - und damit die höchste Empfindlichkeit - werden daher erzielt, wenn zwei, mehrere oder alle diese Parameter in einer multidimensionalen Auswertung gleichzeitig zur Diskriminierung herangezogen werden, wie es z. B. in [Herten, D.P.; Tinnefeld, P.; Sauer, M.; Appl. Phys. B (2000), Band 71, Seiten 765-771] beschrieben ist.

Ebenso ist zusätzlich das in der DE 199 52 160 A1 beschriebene Verfahren nutzbar, bei dem das farbstoffmarkierte Epitop eine andere Nettoladung als der Epitop/Antikörperkomplex hat und daher mit Hilfe eines äußeren elektrischen Feldes wenige gebundene Epitope von einem großen Überschuss an freien Epitopen getrennt werden können.

Im Folgenden wird die Erfindung anhand von Ausführungsbeispielen näher erläutert, die in den Figuren schematisch dargestellt sind. Gleiche Bezugsziffern in den einzelnen Figuren bezeichnen dabei gleiche Elemente. Im Einzelnen zeigt:
- Fig. 1: die Struktur des Oxazins MR121;
- Fig. 2: eine schematische Darstellung der möglichen Zustandsänderungen beim photoinduzierten Elektronentransfer;
- Fig. 3: eine schematische Darstellung der Fluoreszenzlöschung und der Aufhebung der Fluoreszenzlöschung; und

- Fig. 4: einen Vergleich der Fluoreszenzintensität von Einzelmolekül-Ereignissen des freien und des gebundenen farbstoffmarkierten Epitops.

Im Folgenden wird als Ausführungsbeispiel ein Test zur Detektion von monoklonalen Antikörpern der Maus gegen menschliches p53-Protein (BP53-12) beschrieben. Diese Antikörper können käuflich erworben werden.

Es wurde festgestellt [Lubin, R.; Schlichtholz, B.; et al.; Cancer Research 1993, Band 53, Seiten 5872-5876.], dass 90 % aller bisher untersuchten p53-Auto-Antikörper am N-terminalen Ende des p53-Proteins binden. Die Sequenz des Proteins lautet, beginnend am N-terminalen Ende
MEEPQSDPSVEPPLSQETFSDLWKLLPENNVLSPLPSQAMDDLMLSPDDIEQWFTEDPGP-DEAPRMPE...... ,
wobei die Buchstabensymbole der Kurzschreibweise der Aminosäuren verwendet wurden, bei der z. B. W für Tryptophan steht. Die fett unterlegten Aminosäuren wurden als Epitope identifiziert. Die Bindung erfolgt hauptsächlich an den zwei Epitop-Regionen: an den Aminosäuren 11-35 und 41-59.

In manchen Fällen bindet das eine Epitop in manchen Fällen das andere Epitop bei menschlichen Serumproben, da jedes Individuum andere Antikörper bildet. Daher wird immer mit beiden Epitopen in einer Mischung gearbeitet.

Insgesamt ist das Protein 393 Aminosäuren lang.

Es werden beide Epitope synthetisiert, z. B mittels eines üblichen automatisierten Peptidsynthesizers.

Der oben erwähnte Antikörper BP53-12 bindet spezifisch an die Aminosäuren SDLWK des Epitops [Bartek, J.; et al.; J. Pathol. 1993, Band 169, Seiten 27-34] [Dolezalova, H.; et al.; Folia Biol Prague 1997, Band 43, Seiten 49-51].

Als Farbstoff wurde MR121, ein Oxazin gewählt, da die Fluoreszenz von MR121 besonders stark durch Tryptophan gelöscht wird.

Die N-terminale Markierung des Epitops mit dem Farbstoff wurde mit der klassischen N-Hydroxi-Succinimidylester (NHS)-Chemie durchgeführt. Die NHS-Ester von MR121 wurden in einer Konzentration von 1 µmol/ml in Dimethylformamid (DMF) gelöst. Das synthetisierte 15-mer Epitop SQETFSDLWKLLPEN wird in DMF in einer Konzentration von einem 1 µmol/ml gelöst. 10 µl der Farbstofflösung wurden mit 100 µl der Epitop-Lösung unter Zugabe von 2 µl Diisopropylethylamin (DIPEA) als Katalysator gemischt. Die Lösung wurde drei Stunden bei Zimmertemperatur im Dunkeln aufbewahrt. Das N-terminale Konjugat wurde mittels Reversphasen-HPLC (Hypersil-ODS-Säule) mit einem linearen Gradienten von 0-75 % Acetonitril in 0,1 Mol/l wässrigem Triethylammonium-Azetat gereinigt. Eine Reaktionsausbeute von etwa 55 % konnte erreicht werden. Die Reinheit der Probe wurde mit Kapillargel-Elektrophorese überprüft.

Die Antikörper BP53-12 werden in einer Konzentration von etwa 10^-6 Mol/l in 100 mMol/l Natriumphosphat-Pufferlösung bei einem pH-Wert von 7,7 zusammen mit den markierten Epitopen in einer zehnfach höheren Konzentration gelöst. Die Konzentration des Epitops wird 10 mal höher gewählt, um durch das Massenwirkungsgesetz noch eine nachweisbare Menge an gebundenen Komplexen zu erreichen. Die Mischung wird bei 4°C über Nacht im Dunkeln gelagert, um einen Gleichgewichtszustand zu erreichen.

Für die eigentliche Vermessung wird die Probenlösung auf etwa 5*10^-11 Mol/l mittels der Natriumphosphat-Pufferlösung verdünnt, um Einzelmolekül-Experimente zu ermöglichen.

Die Probe wird anschließend vorzugsweise auf einen Objektträger mit einer konkaven Vertiefung übertragen und durch ein Deckglas abgedeckt. Die Probe wird vorzugsweise mit Hilfe eines inversen Mikroskops untersucht. Die Anregung der Probe erfolgt mittels eines Lasers, bevorzugt eines gepulsten Halbleiterlasers mit einer Wellenlänge von 635 nm und einer Pulsrate von 64 MHz und einer Pulsbreite von 200 ps bei einer mittleren Leistung von 250 µW an der Probe.

Das inverse Mikroskop ist in bekannter Weise als konfokaler optischer Aufbau ausgeführt [Sauer, M; et al.; Bioimaging 1998, Band 6, Seiten 14-24] [Sauer, M.; et al.; Phys. Chem. Chem. Phys. 1999, Band 1, Seiten 2471-2477], um das Hintergrundsignal weiter zu reduzieren und damit die Empfindlichkeit so weit zu steigern, dass Einzelmolekül-Events beobachtet werden können. Der Durchmesser des Fokus und damit des Beobachtungsvolumens beträgt etwa 1 µm. Einzelne Moleküle treten aufgrund der Brownschen Molekularbewegung in den Fokus ein und wieder aus. Diese Durchtritte von einzelnen Molekülen können mit dem konfokalen Mikroskop als Einzelmolekül-Events (Bursts) beobachtet werden.

Für das inverse Mikroskop wird ein Öl-Immersions-Objektiv mit 100-facher Vergrößerung und einer numerischen Apertur von 1,4 verwendet. Dadurch ergibt sich eine hohe Sammeleffizienz für das von der Probe emittierte Licht. Das gesammelte Licht wird mit einem Bandpassfilter um 680 nm mit einer Halbwertsbreite des Filters von etwa 50 nm gefiltert und über ein Pinhole auf eine Avalanche-Photodiode geleitet, die in der Lage ist, einzelne Photonen mit hoher zeitlicher Auflösung nachzuweisen.

Im bevorzugten Ausführungsbeispiel ist der Detektor an eine Auswerteelektronik angeschlossen, die neben der Beobachtung des Durchtritts einzelner Moleküle durch den Fokus auch zeitkorreliertes Einzelphotonenzählen ermöglicht, d. h. die es ermöglicht, den zeitlichen Abstand zwischen dem Aussenden eines Lichtpulses durch den Laser und dem Zeitpunkt der Detektion des Photons zu bestimmen. Derartige elektronische Komponenten können käuflich erworben werden. Eine Möglichkeit, eine derartige Elektronik zu realisieren, ist in der WO 98/23941 beschrieben.

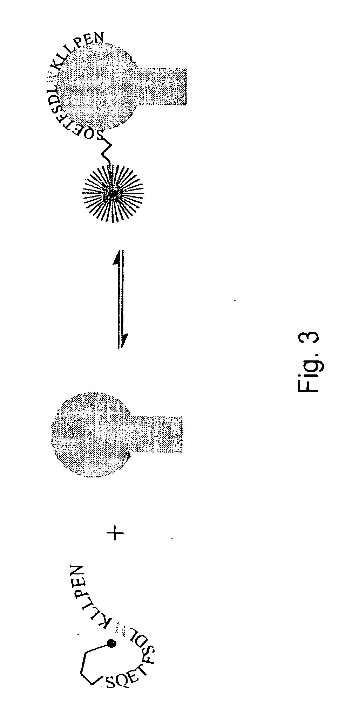

Wie in Fig. 3 schematisch dargestellt, nimmt das mit dem Farbstoff markierte Epitop, wenn es sich frei in der Lösung befindet, eine Konformation ein, bei der der Farbstoff in räumliche Nähe zum Tryptophan-Rest W kommt. Dadurch kommt es zur erwähnten Fluoreszenz-Löschung. Kommt es zu einer Bindung zwischen dem farbstoffmarkierten Epitop und dem Antikörper, so wird das Epitop durch die Bindung mit dem Antikörper in eine bestimmte Konformation gezwungen. In dieser Konformation wird die räumliche Nähe zwischen dem Farbstoff und dem Tryptophan-Rest W aufgelöst. Die Fluoreszenz des Farbstoffs wird dann nicht mehr durch den Tryptophan-Rest W gelöscht. Es kommt somit zu einem Anstieg der Fluoreszenz des Farbstoffs

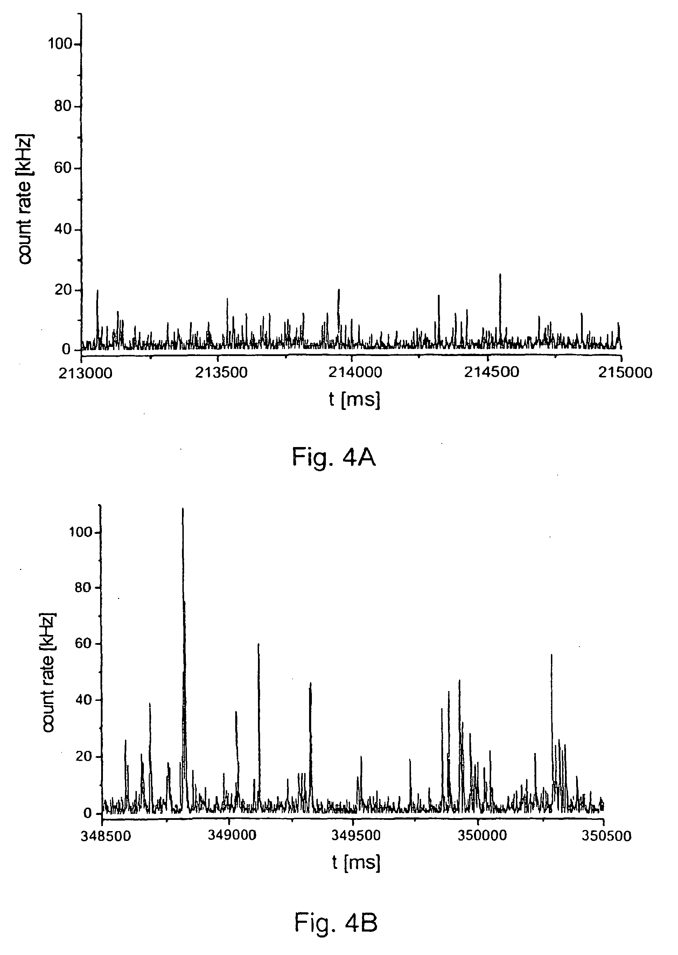

In Fig. 4A und 4B zeigt sich dieser Effekt im Experiment. Fig. 4A zeigt die Zählrate (count rate) in kHz der detektierten Photonen des Detektors des konfokalen Mikroskops, wenn eine Lösung des gefärbten Epitops bei einer Konzentration von 5*10^-11 Mol/l als Probe beobachtet wird. Fig. 4B zeigt den gleichen Graphen für den Fall, dass zusätzlich der Antikörper BP53-12 in hundertfachem Überschuss zugegeben wurde. Durch die Bindung des gefärbten Epitops an den Antikörper ist die Fluoreszenz des Farbstoffs MR121 deutlich erhöht. Setzt man eine Schwelle von z. B. 25 detektierten Photonen pro Millisekunde, entsprechend einer Zählrate von 25 kHz, so werden fast ausschließlich gebundene Epitope und damit Antikörper erkannt.

Ein Vergleich mit dem herkömmlichen ELISA-Test an relevanten Patienten-Proben ergab eine ebenso hohe Empfindlichkeit, nämlich 10^-11 bis 10^-12 Mol/l Antikörper. Die Empfindlichkeit des beschriebenen Verfahrens wurde dabei noch nicht durch Ausnutzung aller Messparameter (Anisotropie, Diffusionszeit, etc., siehe oben) optimiert. Das beschriebene neue Verfahren ist aber wesentlich schneller.

Im Rahmen der Erfindung sind zahlreiche Abwandlungen und Weiterbildungen der beschriebenen Ausführungsbeispiele verwirklichbar.

Das beschriebene Nachweisverfahren ist ideal für den Einsatz in Mikrotiterplatten in Verbindung mit einem Scantisch geeignet, der die einzelnen Probenreservoirs automatisch nach einer Inkubationszeit mit der Serumprobe von ca. 1 h anfährt. Das beschriebene Verfahren ist auch auf andere Auto-Antikörper-Bestimmungen bzw. allgemein auf Nachweise von Antikörpern übertragbar.

### SEQUENCE LISTING

<110> Sauer, Markus
   Wolfrum, Jürgen
<120> Nachweis humaner Auto-Antikörper
<130> WFPT01148
<160> 1
<170> Patent In version 3.1
<210> 1
   <211> 68
   <212> PRT
   <213> human p53 protein
<400> 1

Met Glu Glu Pro Gln Ser Asp Pro Ser Val Glu Pro Pro Leu Ser Gln
1               5                   10                  15

Glu Thr Phe Ser Asp Leu Trp Lys Leu Leu Pro Glu Asn Asn Val Leu
            20                  25                  30

Ser Pro Leu Pro Ser Gln Ala Met Asp Asp Leu Met Leu Ser Pro Asp
        35                  40                  45

Asp Ile Glu Gln Trp Phe Thr Glu Asp Pro Gly Pro Asp Glu Ala Pro
    50                  55                  60

Arg Met Pro Glu
65

### Individual Applicant

Street : Hedwig-Jochmus-Str. 12
City: Heidelberg
State:
Country: Germany
PostalCode: 68124
PhoneNumber :
FaxNumber :
EmailAddress :
<110> LastName : Sauer
   <110> FirstName : Markus
   <110> MiddleInitial :
   <110> Suffix :

### Individual Applicant

Street : Südring 2
City : Rosdorf-Obernjesa
State :
Country : Germany
PostalCode : 37124
PhoneNumber :
FaxNumber :
EmailAddress :
<110> LastName : Wolfrum
   <110> FirstName : Jürgen
   <110> MiddleInitial :
   <110> Suffix:

### Application Project

<120> Title : Nachweis humaner Auto-Antikörper
   <130> AppFileReference : WFPT01148
   <140> CurrentAppNumber :
   <141> CurrentFilingDate :

### Sequence

<213> OrganismName : human p53 protein
   <400> PreSequenceString : 
MEEPQSDPSV EPPLSQETFS DLWKLLPENN VLSPLPSQAM DDLMLSPDDI EQWFTEDPGP
60

DEAPRMPE
68
<212> Type : PRT
   <211> Length : 68
   SequenceName : BP53-12
   SequenceDescription :

## Patentansprüche

1. Verfahren zum Nachweis eines Antikörpers in einer Lösung,
a) wobei der nachzuweisende Antikörper derart gewählt wird, dass er an mindestens ein Epitop eines Antigens binden kann;
b) wobei ein Epitop und ein Fluorophor derart gewählt werden, dass das Epitop mindestens einen Tryptophan-Rest (Quencher) aufweist, der die Lumineszenz des Fluorophors bei hinreichender räumlicher Nähe zwischen dem Fluorophor und dem Tryptophan-Rest quenchen kann;
c) wobei das Epitop mit dem Fluorophor markiert wird;
d) wobei der Antikörper und das markierte Epitop in der Lösung in Kontakt gebracht werden;
e) wobei die Lumineszenz der Lösung in einem Detektionsvolumen beobachtet wird; und
f) wobei eine Änderung der Lumineszenz nach dem In-Kontakt-Bringen von Antikörper und markiertem Epitop als Hinweis für das Vorhandensein des Antikörpers gewertet wird.

2. Verfahren nach dem vorhergehenden Anspruch,
**dadurch gekennzeichnet,**
**dass** die Größe des Detektionsvolumens und die Konzentration der Epitope in der Lösung so aufeinander abgestimmt werden, dass sich im Mittel weniger als ein Epitop im Detektionsvolumen befindet.

3. Verfahren nach einem der vorhergehenden Ansprüche,
**dadurch gekennzeichnet,**
**dass** aus der Lumineszenz der Lösung im Detektionsvolumen spektroskopische Parameter abgeleitet werden; und
**dass** die spektroskopischen Parameter zur Diskriminierung zwischen dem Signal eines freien farbstoffmarkierten Epitops und dem Signal eines farbstoffmarkierten Epitops das an den Antikörper gebunden ist, verwendet werden.

## Claims

1. Process for detecting an antibody in a solution,
a) wherein the antibody to be detected is chosen so as to be able to bind to at least one epitope of an antigen;
b) wherein an epitope and a fluorophore are chosen so as for the epitope to have at least one tryptophan residue (quencher) capable of quenching the luminescence of the fluorophore if the fluorophore and the tryptophan residue are in sufficiently close proximity to one another;
c) wherein the epitope is labelled with the fluorophore;
d) wherein the antibody and the labelled epitope are contacted in the solution;
e) wherein the luminescence of the solution is observed in a detection volume; and
f) wherein a change in luminescence after the antibody and the labelled epitope have been contacted is considered an indication of the presence of the antibody.

2. Process according to the preceding claim,
**characterized in that**
the size of the detection volume and the concentration of the epitopes in the solution are adjusted to one another in such a way that the detection volume contains on average less than one epitope.

3. Process according to either of the preceding claims,
**characterized in that**
spectrometric parameters are derived from the luminescence of the solution in the detection volume; and
the spectrometric parameters are used for discriminating between the signal of a free, dye-labelled epitope and the signal of a dye-labelled epitope bound to the antibody,

## Revendications

1. Procédé d'identification d'un anticorps dans une solution, et dans lequel
a) l'anticorps à identifier est choisi de manière qu'il puisse lier au moins un épitope d'un antigène,
b) un épitope et un fluorophore sont choisis de manière que l'épitope présente au moins un reste tryptophane (désactiveur) capable d'éteindre la luminescence du fluorophore pour une proximité spatiale suffisante entre le fluorophore et le reste tryptophane,
c) l'épitope est marqué par le fluorophore,
d) l'anticorps et l'épitope marqué sont mis en contact dans la solution,
e) la luminescence de la solution est observée dans un volume de détection, et
f) une modification de la luminescence après la mise en contact de l'anticorps et de l'épitope marqué est considérée comme une preuve de la présence de l'anticorps.

2. Procédé suivant la revendication qui précède, **caractérisé en ce que** la grandeur du volume de détection et la concentration de l'épitope dans la solution sont coordonnées de manière qu'il se trouve en moyenne moins d'un épitope dans le volume de détection,

3. Procédé suivant l'une quelconque des revendications qui précèdent, **caractérisé en ce que** l'on dérive de la luminescence de la solution dans le volume de détection des paramètres spectroscopiques, et que les paramètres spectroscopiques sont utilisés pour la discrimination entre le signal d'un épitope libre marqué par un colorant et le signal d'un épitope libre marqué par un colorant et lié aux anticorps;
